# FASCICULE DE BREVET EUROPEEN

(11) **EP 3 982 889 B1**
(45) Date de publication et mention de la délivrance du brevet: **30.10.2024**
(21) Numéro de dépôt: 20742506.7
(22) Date de dépôt: 15.07.2020
(51) Int. Cl.: A61F 5/01, A61F 5/14, A61F 13/06

(54) **DISPOSITIF DE PROTECTION DU CAPITON PLANTAIRE**
VORRICHTUNG ZUM SCHUTZ DER FUSSSOHLENPOLSTERUNG
DEVICE FOR PROTECTING THE PLANTAR PADDING

(30) Priorité: 18.07.2019 FR 1908129
(43) Date de publication de la demande: 20.04.2022
(73) Titulaire: Millet Innovation, 26270 Loriol sur Drome (FR)
(72) Inventeur: LAURENT, Hugo, 26200 Montélimar (FR); FONTAINE, Thierry, 26740 Marsanne (FR); TREPIER-LE BELLER, Maria-Luisa, 4 allée Jean Giono 26800 Portes les Valence (FR); MARTIN, Océane, 26270 Loriol Sur Drome (FR)
(74) Mandataire: Marchand, André
(86) Numéro de dépôt international: PCT/IB2020/056647
(87) Numéro de publication internationale: WO 2021/009691

(56) Documents cités:
- FR-A1- 2 957 246
- FR-A1- 3 031 037
- US-A1- 2018 200 099

## Description

La présente invention concerne un dispositif de protection du pied notamment pour répartir la pression sur l'avant de la plante du pied.

Le pied possède une couche protectrice appelée "capiton plantaire", capable de supporter jusqu'à huit fois le poids du corps. Le capiton plantaire permet de répartir la "charge" mécanique imposée par le poids du corps sur les métatarsiens. Or, de nombreuses personnes souffrent d'échauffement, de durillons ou de douleurs liés à une usure inévitable avec l'âge du capiton plantaire naturel qui ne joue alors plus aussi efficacement son rôle de répartition de charge. En vieillissant, le capiton plantaire a tendance à s'amincir et durcir. Le pied peut également être atteint d'affections cutanées (irritations, fendillements, verrues plantaires...).

Pour soulager ou prévenir ces algies et/ou affections, il est connu de placer sous les têtes métatarsiennes et/ou sous les articulations métatarso-phalangiennes un coussinet dans un matériau viscoélastique choisi pour répartir les charges, notamment pour d'éviter la formation d'hyperkératoses locales, généralement appelées durillon. Pour réaliser un tel coussinet, il est également connu d'utiliser une plaquette à base de gel polymère tel que du gel de silicone ou à base d'hydrogel pour assurer une fonction de substitut au capiton plantaire. Ainsi, une telle plaquette, de quelques centimètres carrés, peut être réalisée à partir d'une composition de gel silicone de type PDMS (polydiméthylsiloxane), par exemple commercialisée par la demanderesse sous l'appellation Epithélium 26^{®}. Le brevet FR 2 712 487 décrit un tel gel de silicone ayant des propriétés se rapprochant de celles du capiton plantaire utilisé pour la prévention de pathologies d'hyper appuis apparaissant essentiellement sur ou sous les pieds.

La présente invention vise particulièrement à protéger le capiton plantaire dans la région des têtes métatarsiennes et à compenser une détérioration de ce dernier en assurant une fonction de répartition de la pression issue de la masse du corps humain sur les têtes métatarsiennes. Cependant, la présence d'un coussinet sous les têtes métatarsiennes peut perturber la posture et l'équilibre de l'utilisateur lors de la marche. En effet, l'équilibre de la position debout résulte de l'intégration en permanence de données venant de trois systèmes récepteurs, à savoir : le système visuel, le système proprioceptif et le système vestibulaire.

Le système visuel (position par rapport à l'environnement) et le système vestibulaire (détection des accélérations rotatoires et linéaires de la tête dans l'espace par un organe de l'oreille) ne devraient pas être perturbés par la présence d'un coussinet sous le pied. Il en va différemment pour le système proprioceptif, car la perception du sol par les mécanorécepteurs cutanés et profonds est atténuée par l'interposition du coussinet. Or la prise en compte des signaux venant de ces capteurs conduit à des réactions musculaires visant à faire disparaître la sensation consciente ou non consciente d'un déséquilibre. Ces réactions musculaires peuvent être à l'origine de pathologies temporaires comme les crampes, ou de plus longue durée comme les tendinites dans toute la cinématique du corps.

La demanderesse a développé et commercialise un coussinet adapté aux seniors ayant un capiton plantaire usé (plus mince et plus dur). Ce coussinet forme une surépaisseur sous le pied supérieure à 3 mm pour une épaisseur de matériau viscoélastique de 2,2 mm. Ce coussinet remplit sa fonction de compensation de l'usure du capiton plantaire. Cependant, le mode de maintien du coussinet sur le pied, l'encombrement et le confort liés au port du coussinet dans un chaussant, méritent d'être améliorés.

Il peut donc être souhaitable de prévoir un coussinet assurant une fonction de répartition de pression, associé à des moyens de maintien aptes à maintenir confortablement le coussinet en place sur le pied, sans entraîner d'instabilité posturale de l'utilisateur. Il peut donc être également souhaitable que l'ensemble du coussinet et des moyens de maintien ne soient pas trop volumineux afin d'être compatible avec le port de chaussures sans introduire de gêne. Il peut être également souhaitable que le coussinet ne puisse pas provoquer de blessures, notamment sur les orteils et des réactions de la peau. Il peut être également souhaitable que l'ensemble du coussinet et des moyens de maintien puissent être utilisés pendant plusieurs mois et puissent être fabriqués facilement.

La demande FR3031037A1 divulgue un dispositif de protection du capiton plantaire d'un pied comprenant un coussinet viscoélastique, prévu pour être maintenu sur la peau de la région plantaire, et couvrant l'ensemble des têtes métatarsiennes et des articulations métatarso-phalangiennes du pied, et une bande élastique fixée au coussinet le long de deux bords transversaux opposés du coussinet et par une patte prévue pour passer entre le gros orteil et l'orteil adjacent du pied, la bande présentant une largeur apte à couvrir entièrement des protubérances latérales interne et externe du pied, formées par les articulations métatarso-phalangiennes du petit orteil et du gros orteil, une partie de la bande destinée à venir en contact avec le dessus du pied, comportant sur ses deux faces des éléments adhérents prévus pour empêcher le dispositif de protection de glisser sur la peau lorsqu'il est disposé sur le pied et sur l'intérieur d'une chaussette enveloppant le pied.

La demande US2018200099A1 concerne un dispositif d'orthèse pour l'avant-pied comportant plusieurs régions configurées de manière à ce que les forces d'impact, de propulsion ou de station debout subies par l'avant-pied de l'utilisateur soient accélérées, décélérées, déplacées, transférées ou réduites, de manière à favoriser la réduction de la douleur ou à traiter autrement les conditions de l'avant-pied. Dans une mise en oeuvre, le dispositif comprend des zones étendues adaptées pour être sous la première et la cinquième têtes métatarsiennes et amortir ou décharger les forces de celles-ci. Dans d'autres variantes, le dispositif comprend un plateau et des régions adjacentes au plateau et inclinées vers le bas à partir de celui-ci, de sorte que le temps pendant lequel les régions douloureuses de l'avant-pied subissent une force, est réduit en faveur du transfert de ces forces vers des régions adjacentes qui ont potentiellement moins besoin de traitement ou de protection contre ces forces.

La demande FR2957246A1 concerne un dispositif orthopédique pour le traitement de l'hallux valgus, comprenant : une partie principale en forme de manchon élastique, destinée à exercer une force de retenue locale sur les métatarses ; une partie distale d'encapsulation du gros orteil ; une bande de liaison entre la partie principale et la partie distale, destinée à être tendue pour exercer sur le gros orteil une force latérale dirigée vers l'intérieur par rapport à l'axe du corps humain ; et un coussinet fixé à la paroi interne du dispositif, créant une épaisseur accrue localisée et destiné à être positionné contre le premier métatarsien du gros orteil, de manière à servir d'ancrage pendant l'application de la force latérale. Le coussinet peut être placé dans différentes positions par rapport au pied, ce qui permet d'ajuster la force de traction sur la bande de liaison. Les forces de retenue et latérales opposées servent à réaligner l'articulation pendant la marche.

Des modes de réalisation de l'invention concernent un dispositif de protection du capiton plantaire d'un pied, comprenant : un coussinet comportant un matériau viscoélastique, prévu pour être maintenu sur la peau de la région plantaire, et couvrir l'ensemble des têtes métatarsiennes et des articulations métatarso-phalangiennes du pied, et une bande élastique fixée à des bords latéraux du coussinet, et comprenant une partie transversale et une partie longitudinale formant une bride prévue pour passer entre le gros orteil et l'orteil adjacent du pied, la partie longitudinale présentant un bord distal fixé à un bord distal du coussinet, la partie transversale présentant un bord distal délimitant avec le coussinet et un bord latéral de la partie longitudinale, une ouverture pour le passage des deuxième à cinquième orteils, le bord distal de la partie transversale et le bord latéral présentant, à plat, sans être étirés, des parties rectilignes formant entre elles un angle compris entre 75 et 100°, le bord latéral présentant une longueur comprise entre 40 et 60% de la longueur du bord distal de la partie transversale, la longueur du bord distal de la partie transversale étant comprise entre 55 et 65% de la longueur de la partie transversale.

Selon un mode de réalisation, le bord distal de la partie longitudinale est fixé au bord distal du coussinet après avoir subi une rotation d'un angle compris entre 80 et 100° dans le plan du coussinet.

Selon un mode de réalisation, le bord distal de la partie longitudinale et le bord distal du coussinet, forment entre eux un angle compris entre 70° et 90°, avant fixation de la partie longitudinale sur le coussinet.

Selon un mode de réalisation, la partie longitudinale présente à son extrémité distale une largeur de 1,5 cm, à + ou - 20% près.

Selon un mode de réalisation, le coussinet comprend une pièce de tissu externe assemblée avec une pièce de tissu interne pour former une poche dans laquelle est logée une plaquette.

Selon un mode de réalisation, la pièce de tissu externe présente une épaisseur comprise entre 0,5 et 0,8 mm, et la pièce de tissu interne présente une épaisseur comprise entre 0,1 et 0,5 mm.

Selon un mode de réalisation, la plaquette est collée sur la pièce de tissu externe.

Selon un mode de réalisation, la plaquette présente une base plane et des excroissances formées sur une face supérieure de la base plane.

Selon un mode de réalisation, la base plane présente une épaisseur comprise entre 1,4 et 2 mm, et les excroissances s'étendent sur une hauteur au-dessus de la base plane, comprise entre 1,3 et 1,7 mm, et présentent un volume total par unité de surface, équivalent à celui d'une couche plane ayant 1/3 de la hauteur, à + ou - 10% près.

Selon un mode de réalisation, les excroissances présentent une forme de calotte sphérique de diamètre égal à 4 mm à + ou - 0,2 mm près, et sont espacées les unes des autres dans des directions à 0°, 60° et 120°, d'une distance de 5 mm à + ou - 0,2 mm près.

Selon un mode de réalisation, le matériau viscoélastique est réalisé à partir d'une composition de gel silicone de type polydiméthylsiloxane.

Des exemples de réalisation de l'invention seront décrits dans ce qui suit, à titre non limitatif en relation avec les figures jointes parmi lesquelles :
[Fig.1] la figure 1 représente schématiquement en vue de dessus un dispositif de protection du capiton plantaire dans la région des têtes métatarsiennes,
[Fig.2] la figure 2 est une vue en coupe du dispositif de protection, dans le plan AA' indiqué sur la figure 1,
[Fig.3] la figure 3 est une vue de dessus du dispositif de protection lors de la fixation d'une bande venant au-dessus du pied, avant la fixation d'une bride,
[Fig.4] la figure 4 est une vue de dessus du dispositif de protection lors de la fixation de la bande, après fixation de la bride,
[Fig.5] la figure 5 représente schématiquement un pied sur lequel est disposé le dispositif de protection,
[Fig.6] la figure 6 est une vue de dessus d'une plaquette logée dans le dispositif de fixation,
[Fig.7] la figure 7 est une vue en coupe de la plaquette de la figure 7, suivant un plan CC' indiqué sur la figure 6,
[Fig.8] la figure 8 représente des courbes illustrant une propriété d'écrasement de la plaquette de la figure 6.

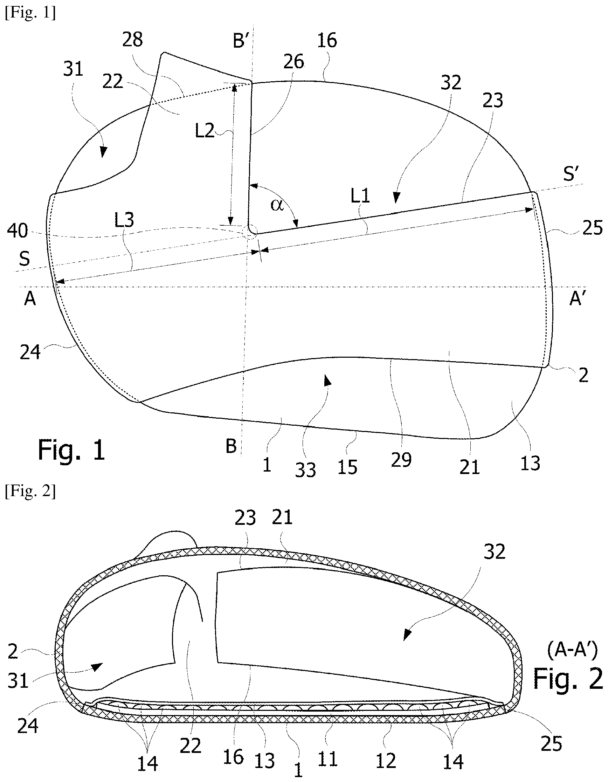

Les figures 1 et 2 représentent un dispositif de protection du pied, selon un mode de réalisation. Le dispositif de protection comprend un coussinet 1 et une bande élastique 2 comprenant une partie transversale 21 fixée au coussinet 1 le long de deux bords latéraux opposés 24, 25 du coussinet 1. L'ensemble du coussinet 1 et de la bande 2 forme ainsi un manchon à deux ouvertures opposées 31-32, 33, ajusté de manière à serrer légèrement l'avant du pied. La bande 2 est également fixée au coussinet 1 par une bride 22 solidaire de la bande 2, et fixée sur un bord longitudinal distal 16 du coussinet. La bride 22 délimite ainsi deux ouvertures distales 31, 32, l'ouverture 31 étant prévue pour le passage du gros orteil du pied, et l'ouverture 32 étant prévue pour le passage des autres orteils. Les fixations latérales et distale de la bande 2 au coussinet 1 sont par exemple réalisées au moyen de coutures.

Selon un mode de réalisation, la bande 2 est réalisée dans un tissu élastique, par exemple un tissu indémaillable, et présente une épaisseur comprise entre 0,5 et 1 mm, par exemple de l'ordre de 0,6 mm.

Selon un mode de réalisation, l'ouverture 32 est délimitée, du côté de la bande 2, par deux bords présentant des parties rectilignes, à savoir un bord transversal 23 de longueur L1, formé par la partie transversale 21, et un bord longitudinal 26 de longueur L2, formé par la bride 22. Les bords 23, 26 sont reliés entre eux par un bord courbe dans une région 40 de la bande 2. Le bord 23 s'étend suivant un axe SS' et le bord 26 s'étend suivant un axe BB', l'axe BB' formant un angle α par rapport à l'axe SS'. Selon un mode de réalisation, l'angle α est compris entre 75 et 100°, le rapport L2/L1 est compris entre 40 et 60%, et de préférence compris entre 45 et 55%, et le bord courbe présente un rayon de courbure inférieur à 1 cm.

Le coussinet 1 comprend une plaquette 11 en un matériau viscoélastique adapté à assurer une fonction de répartition de pression (figure 2). Selon un mode de réalisation, la plaquette 11 est logée dans une poche formée par une pièce de tissu externe 12 assemblée avec une pièce de tissu interne 13. La forme et les dimensions de la poche peuvent être ajustées précisément à celles de la plaquette 11. La plaquette 11 peut être collée sur toute sa surface sur l'une des deux pièces de tissu 12, 13, par exemple la pièce de tissu externe 12. Les pièces de tissu externe 12 et interne 13 peuvent être élastiques et présenter une épaisseur comprise entre 0,5 et 0,8 mm, par exemple de l'ordre de 0,6 mm. Selon un mode de réalisation, la pièce de tissu interne 13 présente une épaisseur plus faible, par exemple comprise entre 0,1 et 0,5 mm, par exemple de l'ordre de 0,2 mm. Les pièces de tissu 12, 13 peuvent être assemblées l'une à l'autre le long de la périphérie de la plaquette 11, par exemple au moyen d'une couture.

Selon un mode de réalisation, la plaquette 11 est réalisée en un gel polymère viscoélastique, par exemple en un gel de silicone. Ainsi, la plaquette 11 peut par exemple être réalisée en PDMS (polydiméthylsiloxane) présentant une dureté shore 00 comprise entre 10 et 40 (équivalent à une dureté shore A de 05), et de préférence 17.

La plaquette 11 peut présenter une épaisseur comprise entre 1,7 et 3,5 mm, par exemple de l'ordre de 1,8 mm. De cette manière l'épaisseur totale du dispositif de protection sous le pied peut atteindre au minimum 2,5 mm. Dans ces conditions d'épaisseur et de dureté de la plaquette 11 et d'épaisseur de son enveloppe en tissu (12, 13), la plaquette 11 est en mesure d'assurer une protection efficace du capiton plantaire, sans perturber la stabilité posturale de l'utilisateur, tout en occupant un espace minimum dans le chaussant.

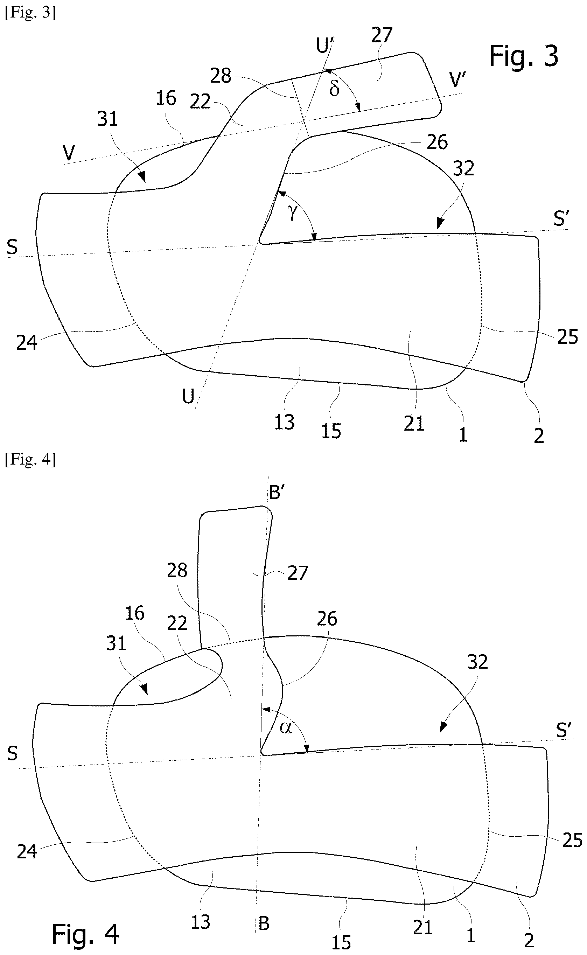

Les figures 3 et 4 montrent la forme de la bande 2, à plat sur la figure 3 et cousue au coussinet 1 sur la figure 4. Sur la figure 3, la bande 2 est posée à plat sur le coussinet 1, puis fixée à ce dernier dans cette position le long des bords latéraux 24, 25 du coussinet 1, (figure 4) sans être étirée, de sorte que la longueur de la bande 2 au repos, après fixation, entre les bords latéraux 24, 25, correspond sensiblement (à 10% près à la largeur du coussinet 1 entre ces bords. Dans cette position, le bord latéral 26 de la bride 22 s'étend dans une direction UU' formant un angle γ par rapport à la direction SS' du bord 23 de la partie transversale 21 côté ouverture 32. La bride 22 présente une partie distale 27 s'étendant sensiblement dans la direction de la partie transversale 21 de la bande 2. La partie distale 27 de la bride 22 s'étend dans une direction VV' formant un angle **δ** avec la direction UU', de sorte que la direction VV' forme un angle **γ** - **δ** avec la direction SS'. Dans l'exemple de la figure 3, l'angle **γ** est compris entre 60° et 85°, et l'angle **γ** - **δ** est de l'ordre de 0 à 20°.

Comme illustré sur les figures 3 et 4, la partie distale 27 de la bride 22 est fixée à plat, sans être étirée, sur le bord distal 16 du coussinet 1 par une ligne de fixation 28 s'étendant sensiblement perpendiculairement (à + ou - 10° près) à la partie distale 27 (direction VV') et s'étendant à partir d'un point de croisement entre le bord latéral 26 de la bride 22 au repos et le bord distal 16 du coussinet 1 (figure 3). La bride 22 est fixée sur le bord distal 16 du coussinet 1, en faisant tourner la bride 22 dans le plan du coussinet 1, de manière à augmenter l'angle **γ** entre les axes SS' et UU' pour atteindre l'angle **α,** et ainsi, pour que l'axe UU' coïncide avec l'axe BB' (figure 1), et en faisant tourner la ligne de fixation 28 sur la bride 22 autour du point de croisement entre le bord latéral 26 de la bride et le bord distal 16 du coussinet 1, pour qu'elle coïncide avec le bord distal 16 du coussinet 1. De cette manière, la bride 22 est fixée sur le coussinet 1 en formant un galbe, maintenant ouverte l'ouverture 31, l'importance de ce galbe étant déterminée par l'amplitude de ces deux rotations. Dans ces conditions, le bord latéral 26 de la bride 22, côté ouverture 32, s'étend suivant l'axe BB' formant l'angle **α** avec l'axe SS'. En considérant que le bord distal 16 à l'emplacement de la fixation de la bride 22 est sensiblement parallèle à l'axe SS', l'angle de rotation de la ligne de fixation 28 est sensiblement égal à 90° - (**γ - δ**), c'est-à-dire compris entre 70 et 90°.

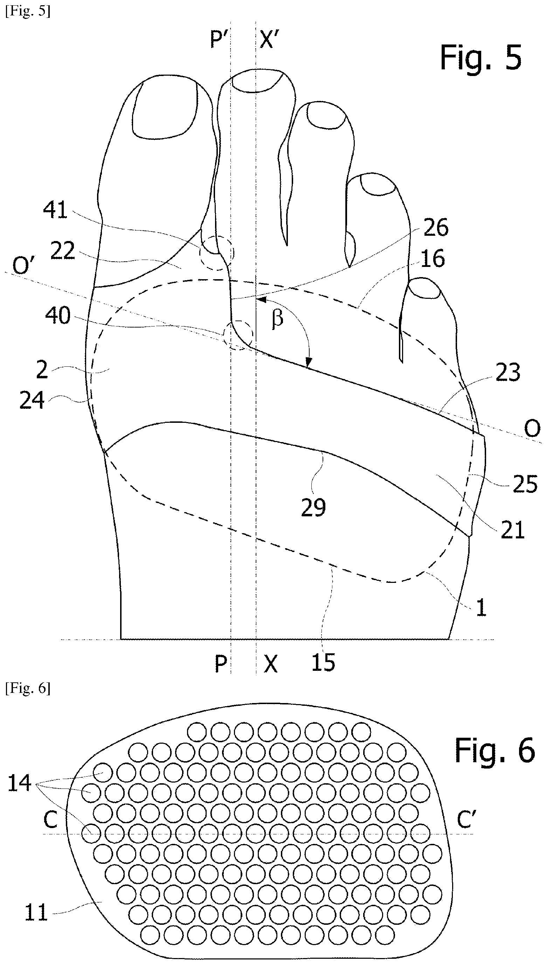

Comme illustré sur la figure 5 montrant le dispositif de protection disposé sur un pied, la forme et les dimensions du coussinet 1 (et donc de la plaquette 11) sont prévues pour couvrir la totalité des têtes métatarsiennes et des articulations métatarso-phalangiennes du pied, et le cas échéant, une partie d'un ou des deux bords latéraux du pied. Le coussinet 1 est prévu pour être disposé sur le pied de manière à ce que son bord distal 16 coïncide sensiblement avec une ligne passant par la base des orteils dans le cas d'un pied de type égyptien. Le bord 23 de l'ouverture 32 appartenant à la partie transversale 21 de la bande 2, s'étend le long d'un axe OO', et le bord 26 de l'ouverture 32 appartenant à la bride 22, s'étend le long d'un axe PP'. Le bord 26 de la bride 22, côté ouverture 32, passe dans la région 41 de la commissure entre le gros orteil et le second orteil. En outre, le galbe entoure le gros orteil.

Lorsque le dispositif de maintien est correctement positionné en rotation autour du pied (autour de l'axe XX'), l'axe PP' est sensiblement parallèle (à + ou - 10° près, selon la morphologie du pied) à l'axe longitudinal XX' du pied, passant par le centre du talon et le milieu de l'extrémité du deuxième orteil. Par ailleurs, lorsque le dispositif de maintien est correctement positionné le long de l'axe XX' du pied, l'axe PP' forme un angle **β** avec l'axe OO' compris entre 100 et 120°, selon la morphologie du pied. Dans l'exemple de la figure 5, l'angle **β** est d'environ 110°.

Lorsque ces conditions de placement correct du dispositif de maintien sur le pied sont respectées, la forme et la disposition de la bride 22 décrites précédemment, permettent d'éviter d'appliquer des tensions entre la région latérale du pied, du côté du petit orteil (ligne de fixation 25), et la région 41 de la commissure entre le gros orteil et le deuxième doigt de pied. Il s'avère que ces tensions auraient tendance à favoriser un déplacement du dispositif vers l'avant du pied et à générer un cisaillement inconfortable sur la commissure entre le gros orteil et le deuxième doigt de pied. L'élimination de ces tensions minimise donc le risque d'un déplacement et donc d'un mauvais positionnement du dispositif de maintien durant son utilisation, et contribue à améliorer le confort du dispositif de maintien autour du gros orteil.

De plus, le galbe donné à la bride 22 entourant le gros orteil, permet au tissu de se coller sur le bord médial de la première articulation métatarso-phalangienne sans mettre de tension importante dans la région 41 de la commissure avec le deuxième doigt de pied. Cette disposition contribue également à améliorer le confort du dispositif de maintien autour du gros orteil.

En comparaison avec une bride sans galbe et avec une longueur L2 réduite ou absente, les caractéristiques de la bride 22 offrent également une capacité d'étirement importante sur la partie de la bride correspondant au passage sur la commissure entre le gros orteil et le deuxième doigt de pied. La présence de la bride 22 est donc moins ressentie par l'utilisateur, ce qui autorise une utilisation de longue durée du dispositif de maintien sans produire de gêne pour l'utilisateur. La durée journalière d'usage du dispositif de maintien se trouve ainsi augmentée.

Selon un mode de réalisation, la bride 22 présente une largeur relativement importante - environ 1,5 cm (à + ou - 20% près) - le long de la couture 28. Cette largeur importante qui est permise par la formation du galbe permet de réduire encore le risque de cisaillement susceptible d'être provoqué par la bride 22 sur la commissure entre les premier et second orteils (région 41).

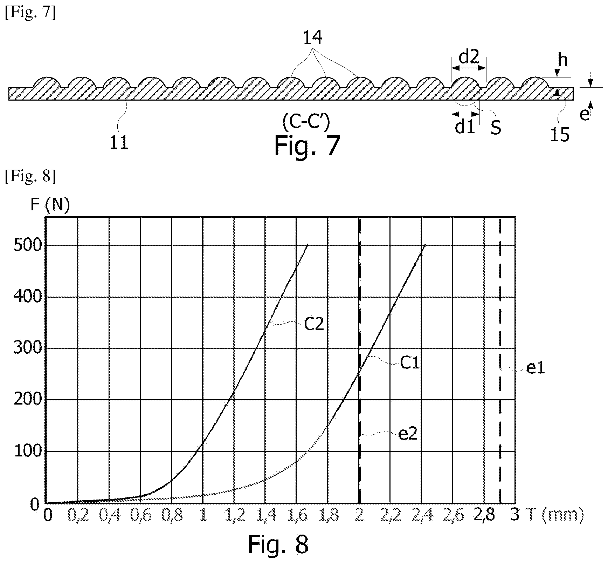

Les figures 6 et 7 représentent la plaquette 11 selon un mode de réalisation. La face supérieure (côté pied de l'utilisateur) de la plaquette 11 présente une base plane 15 d'épaisseur e sur laquelle sont réparties des excroissances 14. Dans l'exemple des figures 6 et 7, les excroissances 14 présentent une même forme de calotte sphérique S de diamètre d1 et une répartition régulière sur la base 15 de la plaquette 11. Les excroissances 14 s'étendent sur une hauteur h au-dessus de la base 15 de la plaquette 11 et sont espacées les unes des autres d'une distance d2 suivant trois directions à 0°, 60° et 120°. Selon un exemple de réalisation, l'épaisseur e est fixée à 1,7 mm (+ ou - 0,3 mm), la hauteur h est fixée à 1,5 mm (+ ou - 0,2 mm), le diamètre d1 des sphères est fixé à 4 mm (+ ou - 0,2 mm), et la distance d2 entre les excroissances 14 est fixée à 5 mm (+ ou - 0,2 mm). Dans ces conditions, le volume de matière au-dessus de la base plane 15 d'épaisseur e se trouve divisé par un facteur de 3 environ, par rapport à une couche d'épaisseur constante ayant la hauteur h. Autrement dit, ce volume correspond à celui d'une couche d'épaisseur constante égale à environ 1/3 de la hauteur h, à + ou - 10% près.

La figure 8 représente des courbes C1, C2 de force F appliquée (en N) en fonction de l'écrasement T (en mm) pour différentes plaquettes. La courbe C1 a été obtenue avec la plaquette 11 (figure 6), et la courbe C2 a été obtenue avec une plaquette plane ayant sensiblement le même volume de matière par unité de surface que la plaquette 11. La figure 8 montre également les épaisseurs initiales e1 (=2.9 mm) de la plaquette 11 et e2 (=2 mm) de l'autre plaquette. Les courbes C1 et C2 présentent chacune trois phases, à savoir une première phase dite de "contact" où l'écrasement du coussinet offre peu de résistance (l'épaisseur du coussinet diminue rapidement en fonction de la force d'écrasement), une seconde phase intermédiaire qui démarre lorsque l'écrasement de la plaquette atteint environ 1 mm pour la plaquette 11 et 0,6 mm pour l'autre plaquette, lorsque la force d'écrasement est comprise entre quelques Newtons et environ 20 N pour les courbes C1 et C2, et une troisième phase, dite de "rigidification", où l'écrasement de la plaquette augmente sensiblement linéairement, plus rapidement en fonction de la force d'écrasement. Durant la phase de rigidification, qui démarre à une force d'environ 200 N pour un écrasement d'environ 1.9 mm pour la courbe C1 et à une force d'environ 100 N pour un écrasement d'environ 0,95 mm pour la courbe C2, les courbes C1 et C2 présentent sensiblement une même pente. Ceci s'explique par le fait que durant cette phase, la plaquette 11 se comporte sensiblement comme une plaque plane.

Par rapport à la courbe C2, la phase intermédiaire de la courbe C1 se trouve donc allongée et se termine avec un écrasement supplémentaire d'environ 0,95 mm. Ceci s'explique par l'écrasement des excroissances 14 jusqu'à ce que l'espace entre ces dernières soit comblé, après être entrées en contact les unes avec les autres, ainsi qu'un écrasement moindre de la base plane 15 de la plaquette 11. Durant cette phase intermédiaire, l'écrasement de la plaquette 11 n'est pas proportionnel à la force appliquée à la plaquette. Cette phase intermédiaire permet d'obtenir un confort "d'accueil", prolongé pour le coussinet 1, au moment où le pied commence à comprimer le coussinet contre le sol. Lorsque la force atteint 500 N durant la phase de rigidification, la plaqette 11 présente une épaisseur d'environ 0,48 mm, et l'autre plaquette, une épaisseur d'environ 0,33 mm. La phase de rigidification ne perturbe pas la stabilité posturale et permet d'éviter un développement local excessif de la couche cornée (kératinisation) en raison de la répartition de pression assurée par le coussinet.

Si l'on compare la plaquette 11 avec une plaquette plane ayant une épaisseur offrant une même capacité de répartition de pression, la plaquette plane doit présenter une épaisseur plus importante que celle e de la base 15 de la plaquette 11 : 2 mm lorsque la base de la plaquette 11 présente une épaisseur de 1,5 mm et une hauteur h des excroissances 14 de 1,4 mm. Les excroissances 14 s'écrasent plus facilement que l'épaisseur de la plaquette plane, en réponse à un chargement et créent une phase de compression des excroissances 14 se produisant avant une phase de compression de la base plane 15, offrant une réponse à la compression similaire à une plaquette plane, avec une épaisseur finale, compte tenu du poids moyen d'une personne adulte (500 N), sensiblement identique (0,48 mm pour la plaquette 11, 0,33 mm pour l'autre plaquette).

Il convient d'observer qu'il se produit un léger emboutissage des excroissances 14 dans les tissus mous du capiton plantaire, qui diminue encore l'épaisseur ressentie sous le pied en comparaison avec une plaquette plane. Il en résulte que la plaquette 11 présente un encombrement potentiel dans la chaussure moins important que la plaquette plane, et donc une amélioration du confort de l'utilisateur et une dégradation moindre de la stabilité lors de la marche. La pénétration des excroissances 14 dans les tissus mous du capiton plantaire contribue également à améliorer le maintien en place du coussinet 1 sous le pied.

Il convient également d'observer que grâce à une diminution de l'épaisseur e de la base 15 de la plaquette 11 par rapport à plaquette plane ayant une épaisseur offrant une même capacité de répartition de pression (même volume de matière par unité de surface), la plaquette 11 épouse davantage l'anatomie de la zone métatarsienne présentant une forme double convexe (convexe longitudinalement et transversalement). En effet, les excroissances 14 n'étant pas liées entre-elles, elles n'augmentent pas la résistance à la flexion de la plaquette 11. C'est donc l'épaisseur e de la base plane 15 qui définit la capacité de résistance à la flexion et donc la capacité de la plaquette à se conformer à la forme du capiton plantaire dans la région des têtes métatarsiennes.

Par ailleurs, le port d'un coussinet sous l'avant-pied engendre l'ajout d'une quantité non négligeable d'un matériau mou dans cette région. Cet ajout modifie l'orientation du pied par rapport au sol (en surélevant l'avant-pied), ainsi que le ressenti du sol lors du chargement du poids du corps en situation statique et pendant le mouvement. Le ressenti du sol perçu par l'utilisateur n'est plus direct, mais retardé dans le temps et peut être modifié par des déformations de cisaillement dans l'épaisseur du coussinet. L'ajout du coussinet a pour conséquence de perturber l'équilibre de l'utilisateur pendant la marche, et de diminuer la capacité de ce dernier à réguler sa posture à l'intérieur de son propre cône de stabilité, en particulier lorsque l'appui s'effectue sur un seul pied durant la phase de propulsion. Il convient ici d'observer que l'équilibre de l'utilisateur peut être perturbé par de nombreux facteurs externes tels que la nature du sol dont la dureté peut varier (par exemple en présence de sable ou d'herbe), des rafales de vent, et la présence de crampons sur les semelles des chaussures. Or, la majorité des personnes ayant besoin de porter une protection sous leurs têtes métatarsiennes sont des seniors qui peuvent déjà avoir une stabilité défaillante ou sur le déclin. Ainsi, le coussinet 1 constitué d'une base plane 15 surmontée d'excroissances 14 présente l'avantage d'être le moins déstabilisateur possible durant les mouvements de marche, en raison d'une diminution d'épaisseur en charge, par rapport à un coussinet ayant la même caractéristique de répartition de pression.

Il apparaîtra clairement à l'homme de l'art que la présente invention est susceptible de diverses variantes de réalisation et diverses applications. En particulier, l'invention n'est pas limitée à une plaquette comportant des excroissances. En effet, la plaquette peut être plane sachant qu'une telle plaquette peut assurer également la fonction de répartition de pression. La plaquette peut être également réalisée dans un matériau viscoélastique pouvant se fixer directement à la bande 2, sans qu'il soit nécessaire d'envelopper la plaquette dans une poche.

La plaquette 11 peut présenter d'autres formes d'excroissances. Les paramètres à considérer pour la couche englobant les excroissances sont la hauteur h des excroissances, la distance d2 entre les excroissances et le taux de remplissage de cette couche, ou bien l'épaisseur appelée dans ce qui suit "épaisseur équivalente" du volume de matière réparti dans les excroissances par unité de surface, lorsque ce volume est réparti dans une couche plane. Selon un mode de réalisation, la hauteur des excroissances est comprise entre 1 et 2 mm et le taux de remplissage de la couche englobant les excroissances, est compris entre 20 et 40%, ou l'épaisseur équivalente des excroissances est comprise entre 20 et 40% de la hauteur des excroissances.

La hauteur h et la forme des excroissances 14, ainsi que la distance séparant les excroissances 14 ne sont pas nécessairement uniformes sur toute la plaquette 11, mais peuvent être définies localement en fonction des besoins, notamment de répartition de pression et d'épaisseur en charge. Ainsi, il peut être constaté que la pression exercée sur le sol par la tête des deux premiers métatarsiens est plus élevée que celle exercée par la tête du cinquième métatarsien. L'épaisseur équivalente des excroissances 14 peut donc être de plus en plus fine le long d'une ligne allant de la tête du premier métatarsien à celle du cinquième métatarsien.

Par ailleurs, le galbe et donc la rotation de la bride 22 pour la fixer au coussinet 1 peuvent être omis, sans affecter le confort du dispositif, en jouant sur la position de la bride 22 par rapport à la bande 2 et sur le coussinet 1.

Dans certaines applications, il peut être souhaitable d'obtenir une meilleure adhérence de la plaquette à la peau. Dans ce cas, la totalité ou seulement une partie de la pièce de tissu interne 13 peut être omise. De même, l'utilisation du dispositif de maintien peut être combinée au port d'une chaussette. Dans ce contexte, il peut être recherché une meilleure adhérence de la plaquette 11 à une chaussette recouvrant le pied. Une partie, notamment centrale, de la pièce de tissu externe 12 peut également être omise.

Il peut également être prévu de noyer une pièce de tissu dans la plaquette 11, cette pièce de tissu étant utilisée notamment pour fixer la bande 2 à la plaquette 11, par exemple au moyen de coutures.

## Revendications

1. Dispositif de protection du capiton plantaire d'un pied, comprenant :
un coussinet (1) comportant un matériau viscoélastique, prévu pour être maintenu sur la peau de la région plantaire, et couvrir l'ensemble des têtes métatarsiennes et des articulations métatarso-phalangiennes du pied, et
une bande élastique (2) fixée à des bords latéraux (24. 25) du coussinet, et comprenant une partie transversale (21) et une partie longitudinale (22) formant une bride prévue pour passer entre le gros orteil et l'orteil adjacent du pied, la partie longitudinale présentant un bord distal (28) fixé à un bord distal (16) du coussinet, la partie transversale présentant un bord distal (23) délimitant avec le coussinet et un bord latéral (26) de la partie longitudinale, une ouverture (32) pour le passage des deuxième à cinquième orteils, **caractérisé en ce que** le bord distal de la partie transversale et le bord latéral présente, à plat, sans être étirés, des parties rectilignes formant entre elles un angle (α) compris entre 75 et 100°, le bord latéral (26) présente une longueur (L2) comprise entre 40 et 60% de la longueur (L1) du bord distal (23) de la partie transversale (21), et la longueur du bord distal de la partie transversale est comprise entre 55 et 65% de la longueur de la partie transversale.

2. Dispositif selon la revendication 1, dans lequel le bord distal de la partie longitudinale (22) est fixé au bord distal (16) du coussinet (1) après avoir subi une rotation d'un angle compris entre 80 et 100° dans le plan du coussinet.

3. Dispositif selon la revendication 1 ou 2, dans lequel le bord distal (28) de la partie longitudinale (22) et le bord distal (16) du coussinet (1), forment entre eux un angle compris entre 70° et 90°, avant fixation de la partie longitudinale sur le coussinet.

4. Dispositif selon l'une des revendications 1 à 3, dans lequel la partie longitudinale (22) présente à son extrémité distale (28) une largeur de 1,5 cm, à + ou - 20% près.

5. Dispositif selon l'une des revendications 1 à 4, dans lequel le coussinet (1) comprend une pièce de tissu externe (12) assemblée avec une pièce de tissu interne (13) pour former une poche dans laquelle est logée une plaquette (11) .

6. Dispositif selon la revendication 5, dans lequel la pièce de tissu externe (12) présente une épaisseur comprise entre 0,5 et 0,8 mm, et la pièce de tissu interne (13) présente une épaisseur comprise entre 0,1 et 0,5 mm.

7. Dispositif selon l'une des revendications 5 et 6, dans lequel la plaquette (11) est collée sur la pièce de tissu externe (12).

8. Dispositif selon l'une des revendications 5 à 7, dans lequel la plaquette (11) présente une base plane (15) et des excroissances (14) formées sur une face supérieure de la base plane.

9. Dispositif selon la revendication 8, dans lequel la base plane (15) présente une épaisseur comprise entre 1,4 et 2 mm, et les excroissances (14) s'étendent sur une hauteur (h) au-dessus de la base plane (15), comprise entre 1,3 et 1,7 mm, et présentent un volume total par unité de surface, équivalent à celui d'une couche plane ayant 1/3 de la hauteur, à + ou - 10% près.

10. Dispositif selon l'une des revendications 8 et 9, dans lequel les excroissances (14) présentent une forme de calotte sphérique de diamètre (d1) égal à 4 mm à + ou - 0,2 mm près, et sont espacées les unes des autres dans des directions à 0°, 60° et 120°, d'une distance (d2) de 5 mm à + ou - 0,2 mm près.

11. Dispositif selon l'une des revendications 1 à 10, dans lequel le matériau viscoélastique est réalisé à partir d'une composition de gel silicone de type polydiméthylsiloxane.

## Patentansprüche

1. Vorrichtung zum Schützen eines Fußballens eines Fußes, umfassend:
ein Kissen (1), das ein viskoelastisches Material aufweist, das zum Gehaltenwerden auf der Haut des Fußsohlenbereichs und zum Bedecken aller Metatarsalköpfchen und Metatarsophalangealgelenke des Fußes vorgesehen ist, und
ein elastisches Band (2), das an seitlichen Rändern (24, 25) des Kissens befestigt ist und umfassend einen Querteil (21) und einen Längsteil (22), der einen Flansch ausbildet, der zum Verlaufen zwischen dem großen Zeh und dem benachbarten Zeh des Fußes vorgesehen ist, wobei der Längsteil einen distalen Rand (28) vorweist, der an einem distalen Rand (16) des Kissens befestigt ist, wobei der Querteil einen distalen Rand (23) vorweist, der mit dem Kissen und einem seitlichen Rand (26) des Längsteils eine Öffnung (32) für den Durchgang des zweiten bis fünften Zehs begrenzt, **dadurch gekennzeichnet, dass** der distale Rand des Querteils und der seitliche Rand, flach, ohne gestreckt zu werden, geradlinige Teile vorweisen, die zwischen sich einen Winkel (α) zwischen 75 und 100° ausbilden, wobei der seitliche Rand (26) eine Länge (L2) zwischen 40 und 60 % der Länge (L1) des distalen Rands (23) des Querteils (21) vorweist, und die Länge des distalen Rands des Querteils zwischen 55 und 65 % der Länge des Querteils beträgt.

2. Vorrichtung nach Anspruch 1, wobei der distale Rand des Längsteils (22) an dem distalen Rand (16) des Kissens (1) befestigt ist, nachdem er in der Ebene des Kissens eine Drehung um einen Winkel zwischen 80 und 100° erfahren hat.

3. Vorrichtung nach Anspruch 1 oder 2, wobei der distale Rand (28) des Längsteils (22) und der distale Rand (16) des Kissens (1) zwischen sich einen Winkel zwischen 70° und 90° ausbilden, bevor das Längsteil an dem Kissen befestigt wird.

4. Vorrichtung nach einem der Ansprüche 1 bis 3, wobei der Längsteil (22) an seinem distalen Ende (28) eine Breite von 1,5 cm auf + oder - 20 % genau vorweist.

5. Vorrichtung nach einem der Ansprüche 1 bis 4, wobei das Kissen (1) ein äußeres Stoffstück (12) umfasst, das mit einem inneren Stoffstück (13) zum Ausbilden einer Tasche zusammengefügt wird, in der eine Platte (11) untergebracht ist.

6. Vorrichtung nach Anspruch 5, wobei das äußere Stoffstück (12) eine Dicke zwischen 0,5 und 0,8 mm vorweist und das innere Stoffstück (13) eine Dicke zwischen 0,1 und 0,5 mm vorweist.

7. Vorrichtung nach einem der Ansprüche 5 und 6, wobei die Platte (11) auf das äußere Stoffstück (12) geklebt ist.

8. Vorrichtung nach einem der Ansprüche 5 bis 7, wobei die Platte (11) eine ebene Basis (15) und Vorsprünge (14) vorweist, die auf einer oberen Fläche der ebenen Basis ausgebildet sind.

9. Vorrichtung nach Anspruch 8, wobei die ebene Basis (15) eine Dicke zwischen 1,4 und 2 mm vorweist und sich die Vorsprünge (14) über eine Höhe (h) über der ebenen Basis (15) zwischen 1,3 und 1,7 mm erstrecken und ein Gesamtvolumen pro Flächeneinheit vorweisen, das dem einer ebenen Schicht entspricht, die 1/3 der Höhe auf + oder - 10 % genau besitzt.

10. Vorrichtung nach einem der Ansprüche 8 und 9, wobei die Vorsprünge (14) eine Form einer Kugelkappe mit einem Durchmesser (d1) von gleich 4 mm auf + oder - 0,2 mm genau vorweisen, und in Richtungen bei 0°, 60° und 120° mit einem Abstand (d2) von 5 mm auf + oder - 0,2 mm genau voneinander beabstandet sind.

11. Vorrichtung nach einem der Ansprüche 1 bis 10, wobei das viskoelastische Material aus einer Silikongelzusammensetzung der Art Polydimethylsiloxan ausgeführt ist.

## Claims

1. A device for protecting a plantar pad of a foot, comprising:
a pad (1) including a viscoelastic material, configured to be held on the skin of the plantar region, and to cover all the metatarsal heads and metatarsophalangeal joints of the foot, and
an elastic strap (2) attached to lateral edges (24, 25) of the pad, and comprising a transverse portion (21) and a longitudinal portion (22) forming a brace configured to pass between a big toe and an adjacent toe of the foot, the longitudinal portion having a distal edge (28) fixed to a distal edge (16) of the pad, the transverse portion having a distal edge (23) delimiting, with the pad and a lateral edge (26) of the longitudinal portion, an aperture (32) for the passage of the second to fifth toes, wherein the distal edge of the transverse portion and the lateral edge have, in a unstretched flat state, rectilinear portions forming between them an angle (α) between 75 and 100°, the lateral edge (26) has a length (L2) between 40 and 60% of the length (L1) of the distal edge (23) of the transverse portion (21), and the length of the distal edge of the transverse portion is between 55 and 65% of the length of the transverse portion.

2. The device of claim 1, wherein the distal edge of the longitudinal portion (22) is attached to the distal edge (16) of the pad (1) after rotating by an angle between 80 and 100° in the plane of the pad.

3. The device of claim 1 or 2, wherein the distal edge (28) of the longitudinal portion (22) and the distal edge (16) of the pad (1) form an angle between 70° and 90° with each other, before fixing the longitudinal portion to the pad.

4. The device of one of claim 1 to 3, wherein the longitudinal portion (22) has at its distal end (28) a width of 1.5 cm ± 20%.

5. The device of one of claim 1 to 4, wherein the pad (1) comprises an outer fabric piece (12) joined with an inner fabric piece (13) to form a pocket in which an insert (11) is housed.

6. The device of claim 5, wherein the outer fabric piece (12) has a thickness between 0.5 and 0.8 mm, and the inner fabric piece (13) has a thickness between 0.1 and 0.5 mm.

7. The device of one of claim 5 and 6, wherein the insert (11) is bonded to the outer fabric piece (12).

8. The device of one of claim 5 to 7, wherein the insert (11) has a planar base (15) and studs (14) formed on an upper side of the planar base.

9. The device of claim 8, wherein the flat base (15) has a thickness between 1.4 and 2 mm, and the studs (14) extend over a height (h) above the flat base (15) between 1.3 and 1.7 mm and have a total volume per unit of area equivalent to that of a flat layer having 1/3 of the height, to within ± 10%.

10. The device of one of claim 8 and 9, wherein the studs (14) have a spherical cap shape with a diameter (d1) of 4 mm ± 0.2 mm, and are spaced apart from each other in 0°, 60° and 120° directions by a distance (d2) of 5 mm to within ± 0.2 mm.

11. The device of one of claim 1 to 10, wherein the viscoelastic material is based on a polydimethylsiloxane silicone gel composition.
